# EUROPÄISCHE PATENTSCHRIFT

(11) **EP 1 161 452 B1**
(45) Veröffentlichungstag und Bekanntmachung des Hinweises auf die Patenterteilung: **15.04.2009**
(21) Anmeldenummer: 00909247.9
(22) Anmeldetag: 24.02.2000
(51) Int. Cl.: C07K 14/62, G01N 33/74, A61K 38/28, A61P 5/48

(54) **KOVALENT VERBRÜCKTE INSULINDIMERE**
COVALENTLY BRIDGED INSULIN DIMERS
DIMERES D'INSULINE PONTES DE MANIERE COVALENTE

(30) Priorität: 24.02.1999 DE 19908041
(43) Veröffentlichungstag der Anmeldung: 12.12.2001
(73) Patentinhaber: Sanofi-Aventis Deutschland GmbH, 65929 Frankfurt am Main (DE)
(72) Erfinder: HÖCKER, Hartwig, D-52076 Aachen (DE); HAVENITH, Chantalle, D-65931 Frankfurt (DE); BRANDENBURG, Dietrich, D-64385 Reichelsheim (DE)
(86) Internationale Anmeldenummer: PCT/EP2000/001530
(87) Internationale Veröffentlichungsnummer: WO 2000/050456

(56) Entgegenhaltungen:
- WO-A-95/05187
- DEPPE, C. ET AL.: "Structure-activity relationship of covalently dimerized insulin derivatives: correlation of partial agonist efficacy with cross-linkage at lysine B29" NAUNYN-SCHMIEDEBERG'S ARCHIVES OF PHARMACOLOGY, Bd. 350, Nr. 2, August 1994 (1994-08), Seiten 213-217, XP000925874
- LEYER, S. ET AL.: "The role of the C-terminus of the insulin B-chain in modulating structural and functional properties of the hormone" INTERNATIONAL JOURNAL OF PEPTIDE & PROTEIN RESEARCH, Bd. 46, Nr. 5, November 1995 (1995-11), Seiten 397-407, XP000530836
- SHOJAEE-MORADIE, F. ET AL.: "Demonstration of a relatively hepatoselective effect of covalent insulin dimers on glucose metabolism in dogs" DIABETOLOGIA, Bd. 38, Nr. 9, September 1995 (1995-09), Seiten 1007-1013, XP000925873 in der Anmeldung erwähnt
- SCHÜTTLER, A. & BRANDENBURG, D.: "Preparation and Properties of Covalently Linked Insulin Dimers" HOPPE-SEYLER'S ZEITSCHRIFT FÜR PHYSIOLOGISCHE CHEMIE, Bd. 363, Nr. 3, März 1982 (1982-03), Seiten 317-330, XP000925886 in der Anmeldung erwähnt

## Beschreibung

Die Erfindung bezieht sich auf neue Insulinanaloga, sowie ein Arzneimittel enthaltend solche Insulinanaloga, ein Verfahren zur Herstellung eines Arzneimittels zur Behandlung von Diabetes sowie ein Verfahren zur Herstellung der insulinanaloga.

Das Proteohormon Insulin wird in den β-Zellen der Langerhansschen Inseln gebildet. Zu seiner wichtigsten physiologischen Wirkung gehört die Senkung des Blutzuckerspiegels. Insulinmangel führt zu dem komplexen Krankheitsbild des Diabetes mellitus (Typ 1), das durch einen abweichenden Glukosestoffwechsel charakterisiert ist.

Zur Behandlung von Diabetes mellitus werden Insulin und Insulinanaloga in pharmazeutischen Zubereitungen eingesetzt. Bei der gebräuchlichsten Therapieform, der Substitutionstherapie, wird Insulin subcutan verabreicht. Als häufigste Nebenwirkung tritt dabei die Hypoglykämie (Unterzuckerung) auf. Trotz der stetigen Entwicklung pharmazeutischer Insulinzubereitungen für die Diabetestherapie wird ständig nach neuen Insulinanaloga gesucht, die vielversprechend im Hinblick auf ihre Wirksamkeit in Kombination mit der Reduzierung von Nebenwirkungen sind. So wurde beispielsweise durch Vertauschen der Aminosäuren Prolin^{B28} und Lysin^{B29} ein schnellwirkendes "Insulin lispro" (EP 0 383 472 B1) entwickelt. Ebenfalls entwickelt wurde durch Fettsäure-Acylierung an der ε-Aminogruppe des Lysins^{B29} ein langwirkendes Insulinderivat (J. Markussen, S. Havelund, P. Kurtzhals, A.A. Andersen, J. Halstrom, E. Hasselager, U.D. Larsen, U. Ribel, L. Schäffer, K. Vad, I. Jonassen, Diabetologia 1996, 39, S. 281-288). Zwar läßt sich der *zeitliche Verlauf* der Insulinwirkung durch derartige Abwandlungen der nativen Struktur des humanen Insulins beeinflussen, wesentliche Probleme sind jedoch noch nicht gelöst: Es gibt noch kein therapeutisch anwendbares Insulinanalogon, welches durch auch nur partielle Gewebsspezifität, insbesondere eine Hepatoselektivität, eine gezieltere, den physiologischen Bedingungen entsprechende Therapie erlaubt. Ebensowenig ist ein Analogon bekannt, das infolge größerer Wirkstärke in geringeren Mengen als Humaninsulin oder tierisches Insulin nativer Struktur eingesetzt werden könnte.

Bei sämtlichen Insulinen, die zur Behandlung von Diabetes eingesetzt werden, handelt es sich immer um monomere Insulinmoleküle mit einer Molmasse um 6000. Alle monomeren Insulinanaloga und -derivate haben sich als partielle oder volle Agonisten des Insulins erwiesen (S. Gammeltoft, Physiol Rev. 1984, 64, S. 1321) und zeigen eine enge Korrelation zwischen Rezeptorbindung und Auslösung des biologischen Signals. Nur in wenigen Fällen, wie beispielsweise bei kovalent verbrückten Insulindimeren, wurde eine Diskrepanz zwischen Rezeptorbindung und biologischer Aktivität beobachtet (A. Schüttler, D. Brandenburg, Hoppe Seyler's Z. Physiol. Chem. 1982, 363, S. 317-330, M. Weiland, C. Brandenburg, D. Brandenburg, H.G. Joost, Proc. Natl. Acad. Sci. USA 1990, 87, S. 1154-1158). Weiterhin haben sich die Insulindimere zur Differenzierung von Insulinrezeptoren in verschiedenen Geweben bewährt (M. Breiner, M. Weiland, W. Becker, D. Müller-Wieland, R. Streicher, M. Fabry, H.G. Joost, Molecular Pharmacology 1993, 44, S. 271-276). Damit sind sie von grundsätzlicher Bedeutung für die Diagnostik in pathotogischen Fällen.

Bei allen bisher beschriebenen Dimeren handelt es sich um die kovalente Verbrückung zweier Insuline in ihrer nativen Länge. Insulindimere sind prinzipiell für die Therapie von besonderem Interesse, da sie im Tierexperiment eine relative Hepatoselektivität zeigen (demonstriert für B1,B1'-Suberoyl-Insulindimer mit nativer Insulinstruktur, F. Shojaee-Moradie, N.C. Jackson, M. Boroujerdi, D. Brandenburg, P.H. Sönksen, R.H. Jones, Diabetologia 1995, 38, S. 1007-1013) und damit eine physiologischere Senkung des Blutzuckerspiegels erlauben als alle zur Zeit in der Diabetestherapie eingesetzten Insuline. Das dort eingesetzte B1,B1'-Suberoyl-Insulindimer hat jedoch eine wesentlich geringere Bioaktivität in vitro als Rezeptorbindung (28,8% gegenüber 157 - 199%, M.A. Tatnell, R.H. Jones, K.P. Willey, A. Schüttler, D. Brandenburg, Biochem. J. 1983, 216, S. 687-694). Das Verhältnis Bioaktivität zu Rezeptorbindung ist also mit 0,15 - 0,18 sehr niedrig. Über eine Anwendung bzw. Weiterentwicklung dieser Befunde in Richtung Diabetestherapie ist uns nichts bekannt.

Deppe et al. (Naunyn-Schmiedeberg's Archives of Pharmacology 350, 1994, S. 213- 217) beschreiben das Verhältnis und Aktivität bei dimerisierten Insulinen, insbesondere in Bezug auf die teilweise agonistische Wirksamkeit bei einer Verbrückung an der Aminosäure Lysin in Position B29. In der internationalen Patentanmeldung WO95/05187 sind hepatoselektive pharmazeutische Wirkstoffe wie Insuline beschrieben, die mit einer molekularen Gruppe wie z.B. einer Thyroxylgruppe kovalent verbunden sind.

Wir haben jetzt neuartige Insulindimere konzipiert und synthetisiert, die aufgrund ihrer Eigenschaften vielversprechend im Hinblick auf eine Lösung der oben genannten Probleme und eine verbesserte Diabetestherapie sind und im folgenden auch als Insulinanaloga bezeichnet werden. Dabei ist gegenüber der bisherigen Therapie mit monomeren Insulinen das einzigartige an unserem Ansatz, daß Dimere von Insulinanaloga bestehend aus zwei gleichen oder verschiedenen Insulinmonomeren, die über eine Brücke kovalent miteinander verbunden sind,
wobei die Insulinmonomere ausgewählt werden aus einer Gruppe enthaltend Humaninsutin und tierische Insuline und Derivate von den vorgenannten Insulinen, und wobei mindestens eines der beiden Insulinmonomere eines Insulinanalogons ein Derivat darstellt; und physiologisch akzeptable Salze davon, verwendet werden. Insbesondere werden solche Insulinanaloga verwendet, bei denen die C-Termini der B-Ketten verkürzt und in Position B26 modifiziert sind. Die Verbrückung der Insulinmonomere kann durch zur Verknüpfung von Proteinen geeigneter Substanzen erfolgen. Solche Substanzen und Verfahren zur Verknüpfung von Proteinen sind seit langem bekannt. Insbesondere besitzen die neuen Dimere von Insulinanaloga eine Brücke, die bevorzugt wischen den N-terminalen Aminogruppen der B-Ketten der beiden Insulinmonomere lokalisiert ist, und die besonders bevorzugt aus einem linearen oder verzweigten bifunktionellen Carbonsäurerest der Form (CRR')ₙ(CO-)₂ gebildet wird, bei dem n, R, R' wie unten bei Formel I ausgeführt, definiert sind. Beispiele für tierische Insuline sind die Insuline vom Schwein, Affen, Rind, und Huhn. Insulinderivate sind Derivate von den genannten Insulinen, welche sich durch Substitution und/oder Deletion wenigstens eines natürlich auftretenden Aminosäurerests und/oder Addition wenigstens eines Aminosäurerests und/oder organischen Rests von dem entsprechenden, ansonsten gleichen natürlich vorkommenden Insulin unterscheiden. Ein Beispiel für ein Insulinderivatmonomer ist Gly(A21), Arg (B31), Arg (B32) Humaninsulin, ein Beispiel für ein erfindungsgemäßes dimeres Insulinanalogon ist B1,B1-Sub-[D-Ala^{B26}]-Des-(B27-B30)-insulin-B26-amid Insulindimer. Insbesondere lassen sich die Dimere von Insulinanaloga mit der allgemeinen Formel I beschreiben: 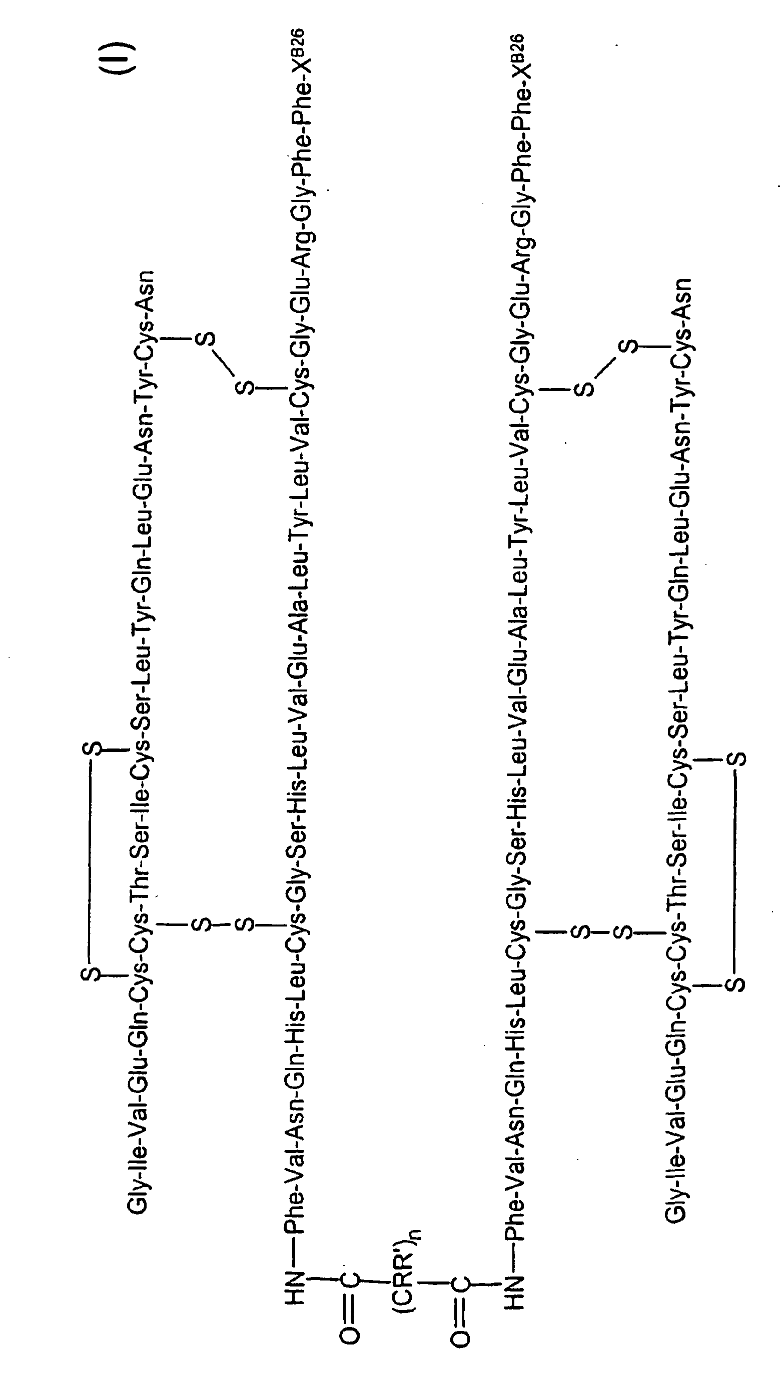 wobei
- X: unabhängig voneinander eine verzweigte oder unverzweigte C₁-C₁₀-Alkylgruppe, einfach oder mehrfach substituierte Arylgruppe, C₁-C₁₀-Alkylgruppe, einfach oder mehrfach substituierte oder unsubstituierte O-Arylgruppe, eine Aminosäure oder ein Derivat davon, oder eine Gruppe der Formel NRR' ist;
- R, R': H, NH₂, ein verzweigter oder unverzweigter C₁-C₁₀-Alkylrest oder ein einfach oder mehrfach substituierte oder unsubstituierte Arylgruppe ist;
- n: 0, 1, 2, .....16 ist.

Ein weiterer Gegenstand der Erfindung sind Dimere von Insulinanaloga der Formel I, wie oben beschrieben, wobei X ein Aminosäurederivat ist, bei welchem die Carbonsäuregruppe amidiert ist.

Ein weiterer Gegenstand der Erfindung sind Dimere von Insulinanaloga der Formel I, wie oben beschrieben, wobei X die Aminosäure Sarcosin ist.

Ein weiterer Gegenstand der Erfindung sind Dimere von Insulinanaloga der Formel I, wie oben beschrieben, wobei die X -Reste in den beiden B-Ketten voneinander verschieden sind.

Ein weiterer Gegenstand der Erfindung sind Dimere von Insulinanaloga der Formel I, wie oben beschrieben, wobei X eine Aminogruppe ist.

Weitere Gegenstände der Erfindung sind ein Arzeimittel und ein Diagnose-Kit enthaltend solche Dimere von Insulinanaloga, ein Verfahren zur Herstellung eines Arzneimittels zur Behandlung von Diabetes sowie ein Verfahren zur Herstellung der Dimere von Insulinanaloga.

Die Dimere von Insulinanaloga werden in bekannter Weise durch Verbrücken von zwei gegebenenfalls partiell geschützten monomeren Molekülen mit der voraktivierten Dicarbonsäure hergestellt (A. Schüttler, D. Brandenburg, Hoppe Seyler's Z. Physiol. Chem. 1982, 363, S. 317-330). Die monomeren Analoga lassen sich mittels enzymkatalysierter Semisynthese oder mit gentechnischen Methoden gewinnen (siehe Erfindungsbeispiele).

Ein weiterer Gegenstand der Erfindung ist demgemäß ein Verfahren zur Herstellung der Dimere von Insulinanaloga wie oben beschrieben, wobei
(a) die monomeren Insulinanaloga mittels enzymkatalysierter Semisynthese oder mittels gentechnischer Methoden gewonnen werden,
(b) die monomeren Insulinanaloga aus Schritt (a) optional durch Schutzgruppen partiell geschützt werden;
(c) die geschützten monomeren Insulinanaloga aus Schritt (b) und/oder die monomeren Insulinanaloga aus Schritt (a) mit einer voraktivierten Dicarbonsäure umgesetz werden, und
(d) die in Schritt (c) erhaltenen Dimere von Insulinanaloga aus dem Reaktionsgemisch isoliert werden.

Verglichen mit Humaninsulin und monomeren Insulinanaloga zeichnen sich die erfindungsgemäßen Dimeren durch besonders hohe Affinität zu Insulinrezeptoren und Superpotenz in vitro aus, letztere bis zu zwanzigfachen der Insulinwirkung.

Im Gegensatz zu den bisher bekannten kovalenten Insulindimeren weisen die neuartigen Dimere sehr hohe Bioaktivitäten auf. Das Verhältnis von Bioaktivität zu Rezeptorbindung ist mindestens 2, teilweise sogar 4 bis 5. Sie sind damit biologisch wesentlich effektiver. Vergleicht man diese Quotienten mit denen des in der Literatur beschriebenen B1,B1'-Suberoyl-Dimeren, ergibt sich mindestens ein Faktor von 11 (0,18 : 2), und maximal von 28.

Die mit den erfindungsgemäßen Insulindimeren erzielten Vorteile bestehen vor allem darin, daß
1. gegenüber Insulin und allen zur Zeit therapeutisch eingesetzten Analoga mit einer relativen Hepatoselektivität und damit einer physiologischeren Wirkungsweise (primärer Wirkort Leber und nicht Peripherie) zu rechnen ist,
2. gegenüber bisher bekannten Insulindimeren erstmalig eine wesentlich verbesserte biologische Effektivität vorhanden ist,
3. die im Vergleich zu Insulin und monomeren Analoga wesentlich erhöhte biologische Aktivität sich bei der Anwendung sehr vorteilhaft erweisen kann, da ein äquivalenter Effekt mit deutlich geringeren Mengen an Wirkstoff zu erreichen wäre.

### Erfindungsbeispiele

### Abkürzungen

- Ac: Acetat (CH₃COO⁻)
- Äq.: Äquivalente
- Alox: Aluminiumoxid (Al₂O₃)
- AS: Aminosäure
- CZE: Kapillarzonenelektrophorese
- DIPEA: N,N-Diisopropylethylamin
- DMF: Dimethylformamid
- DMSO: Dimethylsulfoxid
- DOI: Desoktapeptidinsulin
- Fmoc: 9-Fluorenylmethoxycarbonyl
- HOBt: 1-Hydroxybenzotriazol
- HPLC: Hochleistungsflüssigchromatographie
- MALDI: Matrix-assisted laser desorption / ionization
- MPLC: Mitteldruckflüssigchromatographie
- Msc: Methylsulfonylethoxycarbonyl
- MW: Molekulargewicht
- NaOH: Natriumhydroxid
- NMM: N-Methylmorpholin
- RP: reversed phase
- ONSu: N-Oxysuccinimid-ester
- Sar: Sarcosin
- Sub: Suberoyl
- TBTU: 2-(1H-Benzotriazol-1-yl)-1,1,3,3-tetramethyluronium tetrafluoroborat
- TEA: Triethylamin
- TFA: Trifluoressigsäure
- TOF: time of flight
- TPCK: Tosyl-L-phenylalanyl-chlormethylketon
- Tris: Tris-(hydroxymethyl)-aminomethan

### Erfindungsbeispiel 1

Synthese von
B1,B1'-Sub-[Sar^{B26}]-Des-(B27-B30)-insulin-B26-amid Insulindimer

### Synthese des Tetrapeptids Gly-Phe-Phe-Sar-NH₂

Die Peptidsynthese erfolgte an einem 4-(2',4'-Dimethoxyphenyl-Fmoc-aminomethyl)-phenoxy Harz unter Verwendung der Fmoc-Schutzgruppentaktik. Die zur Kupplung verwendeten Fmoc-Aminosäureester wurden nach der TBTU/HOBt-Methode gebildet und im 3-molaren Überschuß, bezogen auf die nominale Harzbeladung, eingesetzt. Die Peptidsynthese erfolgte nach folgendem Syntheseprotokoll:

| Nr. | Operation | Reagenzien / Lösungsmittel | Zeitdauer | Wiederholungen |
|---|---|---|---|---|
| 1 | Anschwellen des | DMF | 1 min. | einmal |
| | Harzes | | | |
| 2 | Abspaltung der | 20% Piperidin | 6 min. | dreimal |
| | Fmoc-Gruppe | in DMF | | |
| 3 | Waschen | DMF | 0,5 min. | dreimal |
| 4 | Waschen | 2-Propanol | 0,5 min. | zweimal |
| 5 | Kaiser Test* | | | |
| 6 | Anschwellen des | DMF | 1 min. | einmal |
| | Harzes | | | |
| 7 | Kupplung der AS | 3 Äq. Fmoc-AS, jeweils 3 Äq. TBTU, HOBt und 4,5 Äq. NMM in 5 ml DMF | 45 min. | einmal |
| 8 | Waschen | DMF | 0,5 min. | dreimal |
| 9 | Waschen | 2-Propanol | 0,5 min. | zweimal |
| 10 | Kaiser Test** | | | |
| 11 | Anschwellen des | DMF | 1 min. | einmal |
| | Harzes | | | |
| 12 | Blockierung nicht | 400 µl Ac₂O und | | einmal |
| | umgesetzter | 200 µl DIPEA in | 10 min. | |
| | Amino-Endgruppen | 5 ml DMF | | |
| 13 | Waschen | DMF | 0,5 min. | |
| 14 | Waschen | 2-Propanol | 0,5 min. | |

| | | | | |
|---|---|---|---|---|
| *) fahre fort, wenn der Test positiv ist **) fahre fort, wenn der Test negativ ist. Ist das Testergebnis allerdings positiv, wiederhole dann die Schritte 6-9. | | | | |

Die Abspaltung des Peptids vom Harz erfolgte acidolytisch durch Zugabe von 10 ml der Abspaltungslösung aus 95% TFA, 4% H₂O und 1% Triethylsilan als Kationenfänger. Nach 2 h Rühren bei Raumtemperatur wurde das Harz abfiltriert und gründlich mit Dichlormethan gewaschen. Das Filtrat wurde bis zur Trockene eingeengt.

Die weitere Reinigung des Peptids erfolgte mittels RP-MPLC Säulenchromatographie mit einem lineraen 2-Propanol Gradienten (0-40% 2-Propanol in jeweils 400 ml 0,07% TFA Start- und Zulaufpuffer). Als stationäre Phase diente Nucleosil 20-C₁₈. Die Flußrate betrug 180-200 ml/h (82,6% Ausbeute).

### Semisynthese von [Sar^{B26}]-Des-(B27-B30)-insulin-B26-amid

Das Insulin mit verkürztem C-Terminus der B-Kette wurde durch enzymatische Kupplung des Tetrapeptids an N^{αA1}-Msc-Des-(B23-B30)-insulin synthetisiert. Dazu mußte zunächst natives Insulin enzymatisch zu DOI abgebaut werden, welches anschließend partiell mit einer Schutzgruppe versehen wurde.

### Synthese von Des-(B23-B30)-insulin

300 mg (51,66 µmol) Insulin werden in 60 ml Reaktionspuffer (0,05 M Tris, 1 mmol CaCl₂) aufgenommen. Nach Einstellen des pH Werts auf 9,5 mit festem Tris wird der proteolytische Abbau durch Zugabe von 16 mg TPCK-behandeltem Trypsin gestartet. Es werden ca. 6 h bei 37°C im Wasserbad inkubiert, wobei die Reaktion mittels RP-HPLC kontrolliert wird. Die Reaktion wird durch Zugabe von 4 ml Eisessig gestoppt und der Reaktionsansatz am Rotationsverdampfer eingeengt. Die Aufarbeitung erfolgt zunächst über eine Sephadex G-25f- und anschließend über eine Sephadex G-50f-Gelfiltration. Das Produkt wird lyophilisiert (73,3% Ausbeute).
MW: 4865

### Synthese von N^{αA1}-(Msc)-Des-(B23-B30)-insulin

300 mg (61,66 µmol) Des-(B23-B30)-insulin werden unter Zusatz von 225 µl TEA in 22,5 ml DMSO gelöst. Unter leichtem Rühren wird eine Lösung von 18 mg (67,86 µmol) Msc-ONSu in 5 ml DMSO hinzugegeben. Nach einer Reaktionszeit von 20 min. wird die Reaktion durch Zugabe von 750 µl Eisessig gestoppt und die Reaktionslösung 16 h bei 4°C gegen entmineralisertes Wasser dialysiert. Das Retentat wird gefriergetrocknet. Zur weiteren Reinigung werden eine lonenaustausch-Chromatographie an SP-Sepharose (pH 3; 350 ml Startpuffer, 350 ml 0,09 M NaCl-Zulaufpuffer) und Entsalzung über Sephadex G-25f durchgeführt. Das Produkt wird lyophilisiert (30,8% Ausbeute).
MW: 5015,16

### Tryptische Kupplung von Gly-Phe-Phe-Sar-NH₂ an N^{αA1}-(Msc)-Des-(B23-B30)-insulin

132,75 mg (300 µmol) Gly-Phe-Phe-Sar-NH₂ und 150,45 mg (30 µmol) N^{αA1}-Msc-Des-(B23-B30)-insulin werden in 2 ml DMF (über Alox gerührt), 2 ml 1,4-Butandiol und 400 µl 0,05 M Ca(CH₃COO)₂-Lösung gelöst bzw. suspendiert. Mit NMM wird der apparente pH auf 6,7-7,0 eingestellt. Anschließend werden 23 mg TPCKbehandeltes Trypsin, gelöst in 100 µl 0,05 M Ca(CH₃COO)₂-Lösung, dem Reaktionsgemisch zugefügt. Während der Reaktionszeit wird der Verlauf der Reaktion mittels RP-HPLC verfolgt und der pH-Wert überprüft und gegebenfalls mit NMM nachgestellt. In 4,5 h läßt sich ein Umsätz von knapp 90% erzielen. Die Reaktion wird durch Zugabe vom 4,5 ml 30%iger Essigsäure gestoppt. Das Enzym, das Peptid und weitere niedermolekulare Substanzen werden mittels einer Sephadex G-50f Gelchromatographie abgetrennt. Nicht umgesetztes Peptid wird anschließend über RP-MPLC gereinigt und wieder zurückgewonnen. Die weitere Reinigung des Insulinderivats erfolgt über präparative RP-HPLC über eine Nucleosil 100-10C₈ (2,0 cm Durchmesser, 25,0 cm Länge mit einer Vorsäule von 5,0 cm (48,6% Ausbeute).
MW: 5290,2

### Synthese von B1,B1'-Sub-[Sar^{B26}]-Des-(B27-B30)-insulin-B26-amid Insulindimer

100 mg (18,9 µmol) N^{αA1}-Msc-[Sar^{B26}]-des-(B27-B30)-insulin-B26-amid werden unter Zusatz von 5,5 Äquivalenten HOBt in 400 µl DMSO, 8,7 µl DMF und 9,5 µl NMM gelöst. Nach 30 min. wird mit 0,6 Äquivalenten Korksäure-bis-ONSu-ester in fester Form versetzt und 8-30 h gerührt. Der gesamte Reaktionsansatz wird unter Zugabe von 300 µl Eisessig in 1,5 ml 10%ige Essigsäure aufgenommen und über Sephadex G-50f chromatographiert. Die Dimerfraktion wird lyophilisiert. Zur Abspaltung der Msc-Gruppen werden 100 mg Msc-geschütztes Protein in 5 ml eines Dioxan/Wasser Gemisches (2/1, v/v) gelöst und auf 0°C gekühlt. Es wird mit 514 µl 2 N NaOH versetzt und 120 s bei 0°C gerührt. Die Reaktion wird durch Zugabe von 2,2 ml Eisessig gestoppt. Der Reaktionsansatz wird über Sephadex G-25f gelchromatographiert und lyophilisiert (11,9% Ausbeute).
Die Charakterisierung der Zwischenprodukte und des Endprodukts erfolgte mittels RP-HPLC, saurer CZE sowie MALDI-TOF Massenspektrometrie (Tabelle 1).

**Tab. 1: Ausbeuten, Reinheiten nach RP-HPLC sowie CZE und Massen bei der Synthese von B1,B1'-Sub-[Sar^{B26}]-Des-(B27-B30)-insulin-B26-amid Insulindimer**

| Derivat | Ausbeute | Reinheit [%] nach | | MW [g/mol] | |
|---|---|---|---|---|---|
| | [%] | RP-HPLC | CZE | kalk. | gem. |
| Gly-Phe-Phe-Sar-NH₂ | 82,6 | 98,5 | >99 | 442,5 | 463,2* |
| A1-Msc-DOI-Gly-Phe-Phe-Sar-NH₂ | 48,6 | 95,6 | >99 | 5441,2 | 5439 |
| B1,B1'-Sub-DOI-Gly-Phe-Phe-Sar-NH₂-Dimer | 14,4 | 90 | >99 | 10.720 | 10.710 |

| | | | | | |
|---|---|---|---|---|---|
| * Addukt mit Natrium (M = 23) | | | | | |

### Erfindungsbeispiel 2

Synthese von
B1,B1'-Sub-[D-Ala^{B26}]-Des-(B27-B30)-insulin-B26-amid Insulindimer

Die Synthese dieses verkürzte Insulindimer erfolgte analog zur der bei Erfindungsbeispiel 1 beschriebenen Synthese mit der Ausnahme, daß das synthetische Tetrapeptid Gly-Phe-Phe-D-Ala-NH₂ verwendet wurde. In Tabelle 2 sind die entsprechenden Ausbeuten, Reinheiten und Massen wiedergegeben.

**Tab. 2: Ausbeuten, Reinheiten nach RP-HPLC sowie CZE und Massen bei der Synthese von B1,B1'-Sub-[D-Ala^{B26}]-Des-(B27-B30)-insulin-B26-amid Insulindimer**

| Derivat | Ausbeute | Reinheit [%] nach | | MW [g/mol] | |
|---|---|---|---|---|---|
| | [%] | RP-HPLC | CZE | kalk. | gem. |
| Gly-Phe-Phe-D-Ala-NH₂ | 61,6 | 99,1 | >99 | 442,5 | 463,2* |
| A1-Msc-DOI-Gly-Phe-Phe-D-Ala-NH₂ | 53,9 | 95,1 | >99 | 5441,2 | 5439,4 |
| B1,B1'-Sub-DOI-Gly-Phe-Phe-D-Ala-NH₂-Dimer | 11,9 | 88,1 | >99 | 10.720 | 10.713 |

| | | | | | |
|---|---|---|---|---|---|
| * Addukt mit Natrium | | | | | |

### Erfindungsbeispiel 3

Synthese von
B1,B1'-Sub-[Glu^{B26}]-Des-(B27-B30)-insulin-B26-amid Insulindimer

Die Synthese dieses verkürzte Insulindimer erfolgte analog zur der bei Erfindungsbeispiel 1 beschriebenen Synthese mit der Ausnahme, daß das synthetische Tetrapeptid Gly-Phe-Phe-Glu-NH₂ verwendet wurde. Tabelle 3 zeigt die entsprechenden Ausbeuten, Reinheiten und Massen.

**Tab. 3: Ausbeuten, Reinheiten nach RP-HPLC sowie CZE und Massen bei der Synthese von B1,B1'-Sub-[Glu^{B26}]-Des-(B27-B30)-insulin-B26-amid Insulindimer**

| Derivat | Ausbeute | Reinheit [%] nach | | MW [g/mol] | |
|---|---|---|---|---|---|
| | [%] | RP-HPLC | CZE | kalk. | gem. |
| Gly-Phe-Phe-Glu-NH₂ | 74,9 | 94,5 | >99 | 499,2 | 498,3 |
| A1-Msc- DOI-Gly-Phe-Phe-Glu-NH₂ | 38,6 | 95,7 | >99 | 5498,2 | 5497,6 |
| B1,B1'-Sub-DOI-Gly-Phe-Phe-Glu-NH₂-Dimer | 15,8 | >99 | >99 | 10.826 | 10.833 |

### Erfindungsbeispiel 4

### Biologische Eigenschaften zu den Erfindungsbeispielen 1-3

Die biologischen Eigenschaften der in den Erfindungsbeispielen 1-3 beschriebenen Dimere B1,B1'-Sub-[Sar, D-Ala oder Glu^{B26}]-Des-(B27-B30)-insulin-B26-amid Dimer wurden einerseits, anhand der Rezeptorbindung andererseits anhand der Bioaktivität in vitro ermittelt.

Die Bestimmung der Rezeptorbindung erfolgte mittels Verdrängungsstudien an IM-9 Lymphozyten. Die relative biologische Aktivität wurde an kultivierten 3T3-L1 Adipozyten in Form des Glukosetransports bestimmt. In Tabelle 4 sind die Bindungsaffinitäten sowie die relativen biologischen Aktivitäten der synthetisierten Insulindimere wiedergegeben.

**Tab. 4: Relative Rezeptorbindung (bestimmt an IM-9 Lymphozyten) sowie relative biologische Aktivitäten (bestimmt an kultivierten 3T3-L1 Adipozyten) sämtlicher insulindimere im Vergleich zu nativem Insulin.**

| Dimer | Rel. Rezeptor-bindung [%] | Rel. biol. Aktivität [%] |
|---|---|---|
| B1,B1'-Sub-[Sar^{B26}]-Des-(B27-B30)-insulin-B26-amid Insulindimer | 412 ± 94,8 | 1957 ± 575 |
| B1,B1'-Sub-[D-Ala^{B26}]-Des-(B27-B30)-insulin-B26-amid Insulindimer | 357 ± 53,6 | 814 ± 184 |
| B1,B1'-Sub-[Glu^{B26}]-Des-(B27-B30)-insulin-B26-amid Insulindimer | 176 ± 45,8 | 817,5 ± 224 |

### Literatur

1. J. Markussen, S. Havelund, P. Kurtzhals, A.A. Andersen, J. Halstrøm, E. Hasselager, U.D. Larsen, U. Ribel, L. Schäffer, K. Vad, I. Jonassen, Diabetologia 1996, 39, S. 281-288.
2. Europäisches Patent EP 0 383 472 B1.
3. S. Gammeltoft, Physiol Rev. 1984, 64, S. 1321.
4. A. Schüttler, D. Brandenburg, Hoppe Seyler's Z. Physiol. Chem. 1982, 363, S. 317-330.
5. M. Weiland, C. Brandenburg, D. Brandenburg, H.G. Joost, Proc. Natl. Acad. Sci. USA 1990, 87, S. 1154-1158.
6. M. Breiner, M. Weiland, W. Becker, D. Müller-Wieland, R. Streicher, M. Fabry, H.G. Joost, Molecular Pharmacology 1993, 44, S. 271-276.
7. F. Shojaee-Moradie, N.C. Jackson, M. Boroujerdi, D. Brandenburg, P.H. Sönksen, R.H. Jones, Diabetologia 1995, 38, S. 1007-1013.
8. M.A. Tatnell, R.H. Jones, K.P. Willey, A. Schüttler, D. Brandenburg, Biochem. J. 1983, 216, S. 687-694.

## Patentansprüche

1. Dimere von Insulinanaloga, **gekennzeichnet durch** die Formel I, 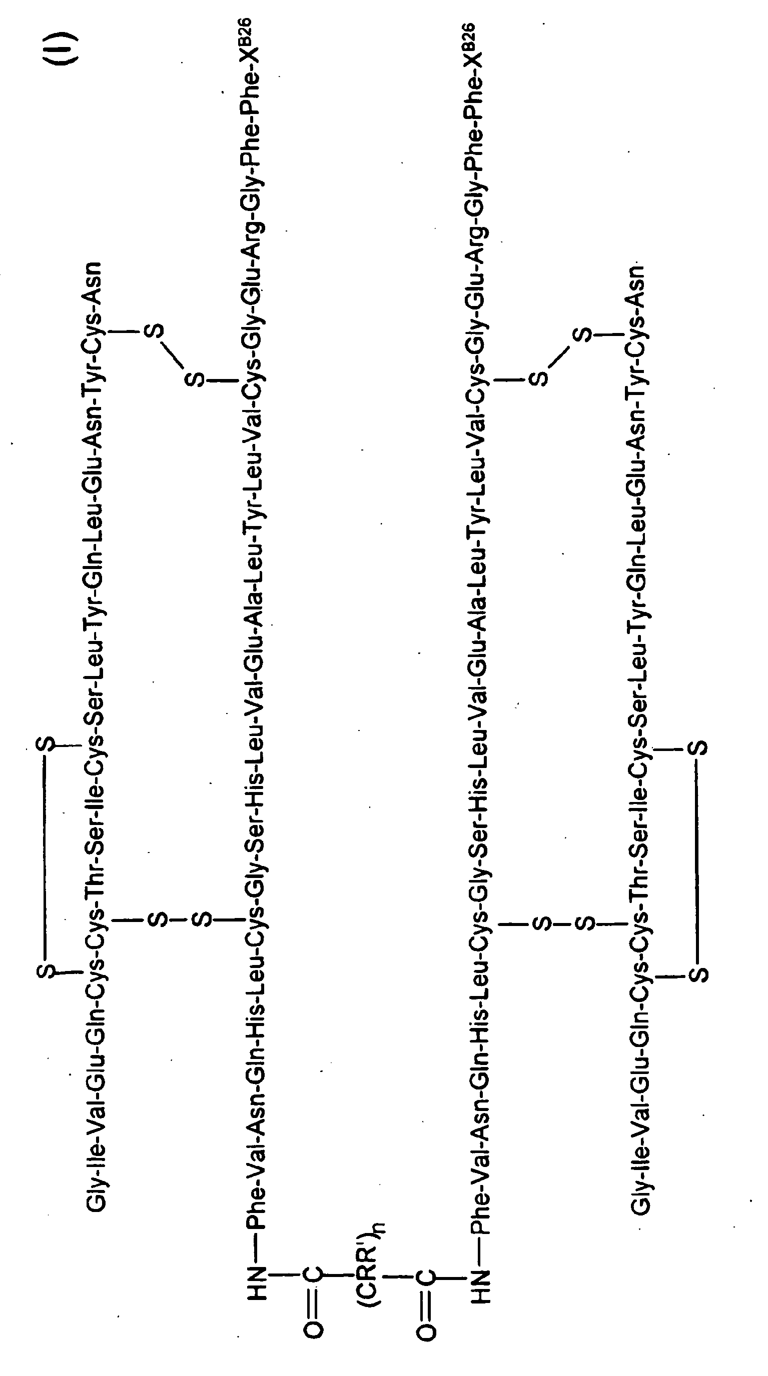 wobei
X unabhängig voneinander eine verzweigte oder unverzweigte C₁-C₁₀-Alkylgruppe, einfach oder mehrfach substituierte Arylgruppe, C₁-C₁₀-Alkylgruppe, einfach oder mehrfach substituierte oder unsubstituierte O-Arylgruppe, eine Aminosäure oder ein Derivat davon, oder eine Gruppe der Formel NRR' ist;
R, R' H, NH₂, ein verzweigter oder unverzweigter C₁-C₁₀-Alkylrest oder ein einfach oder mehrfach substituierte oder unsubstituierte Arylgruppe ist;
n 0,1, 2, .....16 ist.

2. Dimere gemäß Anspruch 1, wobei X eine Aminosäure ist, bei welcher die Carbonsäuregruppe amidiert ist.

3. Dimere gemäß Anspruch 2, wobei X die Aminosäure Sarcosin ist.

4. Dimere gemäß einem oder mehreren der Ansprüche 1 oder 2, wobei die X -Reste in den beiden B-Ketten voneinander verschieden sind.

5. Dimere gemäß einem oder mehreren der Ansprüche 1 oder 2, wobei X eine Aminogruppe ist.

6. B1,B1-Sub-[Sar^{B26}]-Des-(B27-B30)-insulin-B26-amid Insulindimer.

7. B1,B1-Sub-[D-Ala^{B26}]-Des-(B27-B30)-insulin-B26-amid Insulindimer.

8. B1,B1-Sub-[Glu^{B26}]-Des-(B27-B30)-insulin-B26-amid Insulindimer.

9. Pharmazeutische Zubereitungen, **dadurch gekennzeichnet, daß** sie ein Dimer nach einem oder mehreren der Ansprüche 1-8 und Zusätze ausgewählt aus der Gruppe enthaltend Zinksalze, Phenol, m-Kresol, Glycerin und Puffersubstanzen, enthalten.

10. Ein Verfahren zur Herstellung eines Arzneimittels zur Behandlung von Diabetes enthaltend ein Dimer nach einem oder mehreren der Ansprüche 1-8.

11. Dimere nach einem oder mehreren der Ansprüche 1-8 zur Verwendung als Arzneimittel.

12. Diagnosekit enthaltend ein oder mehrere Dimere nach einem oder mehreren der Ansprüche 1-8.

13. Verfahren zur Herstellung der Dimere gemäß einem oder mehreren der Ansprüche 1-8, wobei
(a) die monomeren Insulinanaloga mittels enzymkatalysierter Semisynthese oder mittels gentechnischer Methoden gewonnen werden,
(b) die monomeren Insulinanaloga aus Schritt (a) optional durch Schutzgruppen partiell geschützt werden;
(c) die geschützten monomeren Insulinanaloga aus Schritt (b) und/oder die monomeren Insulinanaloga aus Schritt (a) mit einer voraktivierten Dicarbonsäure umgesetz werden, und
(d) die in Schritt (c) erhaltenen Dimere aus dem Reaktionsgemisch isoliert werden.

## Claims

1. A dimer of insulin analogues, **characterized by** formula I, 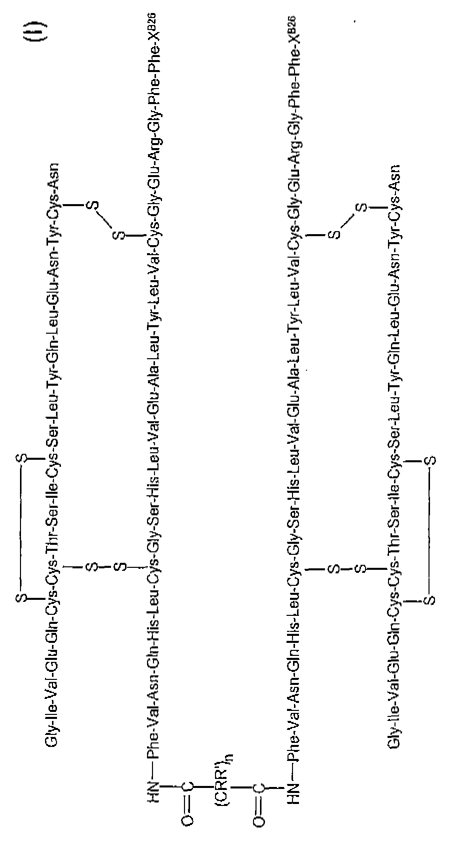 where
X is, independently of one another, a branched or unbranched C₁-C₁₀-alkyl group, mono- or polysubstituted aryl group, C₁-C₁₀-alkyl group, mono- or polysubstituted or unsubstituted O-aryl group, an amino acid or a derivative thereof, or a group of the formula NRR';
R, R' is H, NH₂, a branched or unbranched C₁-C₁₀-alkyl radical or mono- or polysubstituted or unsubstituted aryl group;
n is 0, 1, 2, ...........16.

2. A dimer as claimed in claim 1, where X is an amino acid in which the carboxylic acid group is amidated.

3. A dimer as claimed in claim 2, where X is the amino acid sarcosine.

4. A dimer as claimed in one or more of claims 1 or 2, where the X residues in the two B chains are different from one another.

5. A dimer as claimed in one or more of claims 1 or 2, where X is an amino group.

6. B1,B1-Sub-[Sar^{B26}]-des-(B27-B30)-insulin-B26-amide insulin dimmer.

7. B1,H1-Sub-[D-Ala^{B26}]-des-(B27-B30)-insulin-B26-amide insulin dimer.

8. S1,B1-Sub-[Glu^{B26}]-des-(B27-B30)-insulin-B26-amide insulin dimer.

9. A pharmaceutical preparation which comprises a dimer as claimed in one or more of claims 1-8 and additions selected from the group comprising zinc salts, phenol, m-cresol, glycerol and buffer substances.

10. A process for producing a pharmaceutical for the treatment of diabetes comprising a dimer as claimed in one or more of claims 1-8.

11. A dimer as claimed in one or more of claims 1-8 for use as pharmaceutical.

12. A diagnostic kit comprising one or more dimers as claimed in one or more of claims 1-8.

13. A process for preparing the dimers as claimed in one or more of claims 1-8, where
(a) the monomeric insulin analogues are obtained by enzyme-catalyzed semisynthesis or by methods of genetic manipulation,
(b) the monomeric insulin analogues from step (a) are optionally partially protected by protective groups;
(c) the protected monomeric insulin analogues from step (b) and/or the monomeric insulin analogues from step (a) are reacted with a preactivated dicarboxylic acid, and
(d) the dimers obtained in step (c) are isolated from the reaction mixture.

## Revendications

1. Dimères d'analogues d'insuline, **caractérisés par** la formule I, 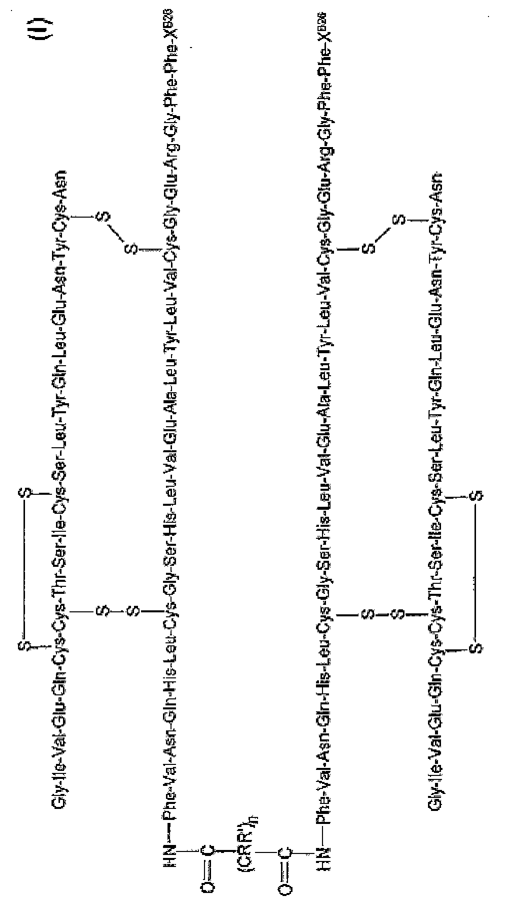 où
X représente, indépendamment l'un de l'autre, un groupe C₁-C₁₀-alkyle ramifié ou non ramifié, un groupe aryle, un groupe C₁-C₁₀-alkyle monosubstitué, polysubstitué, un groupe O-aryle monosubstitué ou polysubstitué ou non substitué, un acide aminé ou un dérivé de celui-ci ou un groupe de formule NRR' ;
R, R' représentent H, NH₂, un radical C₁-C₁₀-alkyle ramifié ou non ramifié ou un groupe aryle monosubstitué, polysubstitué ou non substitué ;
n vaut 0, 1, 2, ..., 16.

2. Dimères selon la revendication 1, où X représente un acide aminé dans lequel le groupe acide carboxylique est amidé.

3. Dimères selon la revendication 2, où X représente l'acide aminé sarcosine.

4. Dimères selon l'une ou plusieurs des revendications 1 ou 2, où les radicaux X dans les deux chaînes B sont différents l'un de l'autre.

5. Dimères selon l'une ou plusieurs des revendications 1 ou 2, où X représente un groupe amino.

6. Dimère d'insuline B1,B1-Sub-[Sar^{B26}]-Des-(B27-B30)-insuline-B26-amide.

7. Dimère d'insuline B1,B1-Sub-[D-Ala^{B26}]-Des-(B27-B30)-insuline-B26-amide.

8. Dimère d'insuline B1,B1-Sub-[Glu^{B26}]-Des-(B27-B30)-insuline-B26-amide.

9. Préparations pharmaceutiques, **caractérisées en ce qu'**elles contiennent un dimère selon l'une ou plusieurs des revendications 1-8 et des additifs choisis dans le groupe contenant les sels de zinc, le phénol, le m-crésol, le glycérol et les substances tampon.

10. Procédé pour la préparation d'un médicament destiné au traitement du diabète contenant un dimère selon l'une ou plusieurs des revendications 1-8.

11. Dimères selon l'une ou plusieurs des revendications 1-8 destinés à une utilisation comme médicament.

12. Kit diagnostique contenant un ou plusieurs dimères selon l'une ou plusieurs des revendications 1-8.

13. Procédé pour la préparation des dimères selon l'une ou plusieurs des revendications 1-8, dans lequel
(a) les analogues monomères d'insuline sont obtenus au moyen d'une semi-synthèse catalysée par des enzymes ou au moyen de procédés de technique génétique,
(b) les analogues monomères d'insuline de l'étape (a) sont éventuellement protégés partiellement par des groupes de protection ;
(c) les analogues monomères d'insuline protégés de l'étape (b) et/ou les analogues monomères d'insuline de l'étape (a) sont transformés avec un acide dicarboxylique activé au préalable, et
(d) les dimères obtenus dans l'étape (c) sont isolés du mélange réactionnel.
